# EUROPEAN PATENT APPLICATION

(11) **EP 2 423 320 A1**
(43) Date of publication of application: **29.02.2012**
(21) Application number: 10767119.0
(22) Date of filing: 22.04.2010
(51) Int. Cl.: C12P 17/18, C12N 15/09

(54) **PROCESS FOR PRODUCTION OF (R)-3-QUINUCLIDINOL**

(30) Priority: 23.04.2009 JP 2009105390
(71) Applicant: Kaneka Corporation, Osaka-shi, Osaka 530-8288 (JP); National University Corporation Nara Institute of Science and Technology, Ikoma-shi, Nara 630-0192 (JP)
(72) Inventor: KAWANO Shigeru, Takasago-shi Hyogo 676-8688 (JP); NISHIHACHIJYO Masakatsu, Takasago-shi Hyogo 676-8688 (JP); YASOHARA Yoshihiko, Takasago-shi Hyogo 676-8688 (JP); ONODERA Keiko, Ikoma-shi Nara 630-0192 (JP)
(74) Representative: Vossius & Partner
(86) International application number: PCT/JP2010/057139
(87) International publication number: WO 2010/123062

(57) **Abstract**

Provided is an efficient and industrial method for producing (R)-3-quinuclidinol which is a compound useful as an intermediate for the production of pharmaceuticals. The method for producing (R)-3-quinuclidinol includes reducing 3-quinuclidinone or a salt thereof in the presence of a polypeptide derived from bacteria of the genus *Burkholderia* or a recombinant organism capable of producing the polypeptide.

## Description

### TECHNICAL FIELD

The present invention relates to a method for producing (R)-3-quinuclidinol. Optically active 3-quinuclidinol is a compound useful as a raw material and an intermediate for synthesizing products such as pharmaceuticals and agricultural chemicals.

### BACKGROUND ART

(R)-3-quinuclidinol is a compound useful as a raw material and an intermediate for synthesizing products such as pharmaceuticals and agricultural chemicals. Examples of the method for producing (R)-3-quinuclidinol include asymmetric reduction of 3-quinuclidinone using microbial cells as a catalyst.

The microbial catalyst used in the method, however, is limited to cells of microorganisms of the genera *Nakazawaea, Candida, Proteus, Rhodotorula, Sporidiobolus, Rhodosporidium, Schizosaccharomyces, Cryptococcus, Trichosporon, Gordonia, Nocardia, Alcaligenes, Corynebacterium, Arthrobacter, Filobasidium, Aureobasidium, Yarrowia, Geotrichum, Tsukamurella, Kurthia, Microbacterium, Kluyveromyces, Acremonium,* and *Mucor* (Patent Documents 1, 2, 3, and 4).

In a reaction using such microbial cells, factors such as impurities derived from the microbial cells or secondary reactions caused by other enzymes contained by the microorganism sometimes complicate isolation of the reaction product. The reaction is also accompanied by problems such as low optical purity of the product and low concentration of the accumulated product.

Further, a method including asymmetric reduction of 3-quinuclidinone using a specific enzyme as a catalyst has been reported. The enzyme used in the method, however, is limited to enzymes derived from plants of the genera *Datura* and *Hyoscyamus* (Patent Document 5), enzymes derived from microorganisms of the genus *Microbacterium* (Patent Document 6), and enzymes derived from microorganisms of the genus *Rhodotorula* (Patent Document 7).

Patent Document 1: JP 10-243795 A
Patent Document 2: Jap 11-196890 A
Patent Document 3: JP 2002-153293 A
Patent Document 4: JP 2000-245495 A
Patent Document 5: JP 2003-230398 A
Patent Document 6: JP 2003-334069 A
Patent Document 7: JP 2007-124922 A

### SUMMARY OF THE INVENTION

The present invention aims to provide an efficient and industrial method for producing (R)-3-quinuclidinol which is useful as an intermediate for pharmaceuticals.

The present inventors have made studies to develop a simple and efficient method for producing (R)-3-quinuclidinol and, as a result of the studies, the inventors have found a novel polypeptide capable of converting 3-quinuclidinone into (R)-3-quinuclidinol from microorganisms of the genus *Burkholderia*, thereby completing the present invention.

That is, the present invention has the following one or more features.

One aspect of the present invention is a method for producing (R)-3-quinuclidinol, including reducing 3-quinuclidinone or a salt thereof in the presence of a polypeptide, an organism capable of producing the polypeptide, or a treated product of the organism, the polypeptide being selected from the group consisting of:
(a1) a polypeptide having an amino acid sequence of SEQ ID NO:1 in the sequence listing;
(a2) polypeptides having an amino acid sequence derived from the amino acid sequence of SEQ ID NO:1 in the sequence listing by substitution, deletion, insertion and/or addition of one or a plurality of amino acid residues and capable of acting on 3-quinuclidinone and NADPH to form (R)-3-quinuclidinol and NADP;
(a3) polypeptides having a sequence identity of at least 85% with the amino acid sequence of SEQ ID NO:1 in the sequence listing and capable of acting on 3-quinuclidinone and NADPH to form (R)-3-quinuclidinol and NADP;
(b1) a polypeptide having an amino acid sequence of SEQ ID NO:3 in the sequence listing;
(b2) polypeptides having an amino acid sequence derived from the amino acid sequence of SEQ ID NO:3 in the sequence listing by substitution, deletion, insertion and/or addition of one or a plurality of amino acid residues and capable of acting on 3-quinuclidinone and NADPH to form (R)-3-quinuclidinol and NADP; and
(b3) polypeptides having a sequence identity of at least 85% with the amino acid sequence of SEQ ID NO:3 in the sequence listing and capable of acting on 3-quinuclidinone and NADPH to form (R)-3-quinuclidinol and NADP.

The organism capable of producing the polypeptide selected from the group consisting of (a1), (a2), (a3), (b1), (b2), and (b3) is preferably a recombinant organism having introduced therein a DNA selected from the group consisting of:
(A1) a DNA of SEQ ID NO:2 in the sequence listing;
(A2) DNAs encoding the polypeptide having the amino acid sequence of SEQ ID NO:1 in the sequence listing;
(A3) DNAs hybridizing with a DNA complementary to the DNA of SEQ ID NO:2 in the sequence listing under stringent conditions and encoding a polypeptide capable of acting on 3-quinuclidinone and NADPH to form (R)-3-quinuclidinol and NADP;
(A4) DNAs having a sequence identity of at least 85% with the base sequence of SEQ ID NO:2 in the sequence listing and encoding a polypeptide capable of acting on 3-quinuclidinone and NADPH to form (R)-3-quinuclidinol and NADP;
(B1) a DNA of SEQ ID NO:4 in the sequence listing;
(B2) DNAs encoding the polypeptide having the amino acid sequence of SEQ ID NO:3 in the sequence listing;
(B3) DNAs hybridizing with a DNA complementary to the DNA of SEQ ID NO:4 in the sequence listing under stringent conditions and encoding a polypeptide capable of acting on 3-quinuclidinone and NADPH to form (R)-3-quinuclidinol and NADP; and
(B4) DNAs having a sequence identity of at least 85% with the base sequence of SEQ ID NO:4 in the sequence listing and encoding a polypeptide capable of acting on 3-quinuclidinone and NADPH to form (R)-3-quinuclidinol and NADP.

The recombinant organism is preferably *Escherichia coli.*

The production method preferably further includes
utilizing a polypeptide capable of regenerating a reduced coenzyme.

The polypeptide is preferably produced by the recombinant organism capable of producing the polypeptide capable of regenerating a reduced coenzyme.

The polypeptide capable of regenerating a reduced coenzyme is preferably a glucose dehydrogenase.

Another aspect of the present invention is a method for producing (R)-3-quinuclidinol, including
reducing 3-quinuclidinone or a salt thereof in the presence of a polypeptide derived from bacteria of the genus *Burkholderia.*

Yet another aspect of the present invention is a method for producing an active pharmaceutical ingredient, including the step of
producing (R)-3-quinuclidinol by reducing 3-quinuclidinone or a salt thereof in the presence of a polypeptide, an organism capable of producing the polypeptide, or a treated product of the organism,
the polypeptide being selected from the group consisting of:
(a1) a polypeptide having an amino acid sequence of SEQ ID NO:1 in the sequence listing;
(a2) polypeptides having an amino acid sequence derived from the amino acid sequence of SEQ ID NO:1 in the sequence listing by substitution, deletion, insertion and/or addition of one or a plurality of amino acid residues and capable of acting on 3-quinuclidinone and NADPH to form (R)-3-quinuclidinol and NADP;
(a3) polypeptides having a sequence identity of at least 85% with the amino acid sequence of SEQ ID NO:1 in the sequence listing and capable of acting on 3-quinuclidinone and NADPH to form (R)-3-quinuclidinol and NADP;
(b1) a polypeptide having an amino acid sequence of SEQ ID NO:3 in the sequence listing;
(b2) polypeptides having an amino acid sequence derived from the amino acid sequence of SEQ ID NO:3 in the sequence listing by substitution, deletion, insertion and/or addition of one or a plurality of amino acid residues and capable of acting on 3-quinuclidinone and NADPH to form (R)-3-quinuclidinol and NADP; and
(b3) polypeptides having a sequence identity of at least 85% with the amino acid sequence of SEQ ID NO:3 in the sequence listing and capable of acting on 3-quinuclidinone and NADPH to form (R)-3-quinuclidinol and NADP.

The active pharmaceutical ingredient is preferably solifenacin.

The method of the present invention allows efficient and industrial production of (R)-3-quinuclidinol in high optical purity.

### MODES FOR CARRYING OUT THE INVENTION

Hereafter, the present invention is explained in detail based on embodiments which, however, are by no means limitative of the scope of the invention.

### Polypeptide derived from bacteria of the genus Burkholderia

The polypeptide used in the present invention can be selected from polypeptides which are derived from bacteria of the genus *Burkholderia* and capable of reducing 3-quinuclidinone to form (R)-3-quinuclidinol. The *Burkholderia* bacteria capable of producing the polypeptide can be selected by, for example, the following method.

The *Burkholderia* bacteria are cultured on an appropriate medium. Cells are collected from the medium and brought into contact with 3-quinuclidinone for the reaction in a buffer solution in the presence of nutrients such as glucose. After the reaction, the reaction mixture is extracted using a solvent or the like, and the extract can be analyzed by gas chromatography or the like to determine the generated amount and optical purity of 3-quinuclidinol.

Usable as the medium for culturing the *Burkholderia* bacteria are ordinary nutrient broths containing carbon and nitrogen sources, inorganic salts, organic nutrients and so forth, provided that the *Burkholderia* bacteria can grow on the medium. The culturing can be performed by, for example, shaking or aerating at a temperature of 25°C to 37°C and a pH of 4 to 8.

The polypeptide used in the present invention can be isolated from the *Burkholderia* bacteria by appropriately combined known protein purification methods. For example, the polypeptide can be isolated as described below.

First, *Burkholderia* bacteria are cultured on an appropriate medium, and bacterial cells are collected from the culture fluid by centrifugation or filtration. The cells obtained are disrupted by a sonicator or a physical means, for example by the use of glass beads, and the cell debris is then removed by centrifugation to give a cell-free extract. The polypeptide used in the present invention is isolated from the cell-free extract by using such techniques as heat treatment, salting out (e.g. precipitation with ammonium sulfate or sodium phosphate), solvent precipitation (fractional protein precipitation with acetone or ethanol, for instance), dialysis, gel filtration chromatography, ion exchange chromatography, reversed phase chromatography and ultrafiltration, applied either singly or in combination.

The reduction activity and stereoselectivity of the isolated polypeptide to 3-quinuclidinone can be evaluated by exposing the polypeptide to be evaluated to 3-quinuclidinone and then analyzing the reaction mixture by gas chromatography or the like. The 3-quinuclidinone reduction activity can also be evaluated by the following simple method.

### [Method for measuring reduction activity to 3-quinuclidinone]

The reduction activity to 3-quinuclidinone was calculated by adding 3-quinuclidinone (10 mM), a coenzyme NADPH (0.25 mM), and a crude enzyme liquid to a 100 mM phosphate buffer (pH 6.5), allowing the reaction to proceed at 30°C for one minute, and determining the rate of decrease in the absorbance at a wavelength of 340 nm. The enzyme activity of oxidizing 1 µmol of NADPH to NADP in one minute under such reaction conditions was defined as 1 U.

The source of the polypeptide used in the present invention is not limited as long as it is derived from *Burkholderia* bacteria. Examples of the *Burkholderia* bacteria include *Burkholderia ambifaria, Burkholderia andropogonis, Burkholderia anthina, Burkholderia* caledonica, *Burkholderia caryophylli, Burkholderia caribensis, Burkholderia cenocepacia, Burkholderia cepacia, Burkholderia dolosa, Burkholderia endofungorum, Burkholderia ferrariae, Burkholderia fungorum, Burkholderia ginsengisoli, Burkholderia gladioli, Burkholderia glathei, Burkholderia glumae, Burkholderia graminis, Burkholderia hospita, Burkholderia kururiensis, Burkholderia multivorans, Burkholderia phenazinium, Burkholderia phenoliruptrix, Burkholderia phymatum, Burkholderia phytofirmans, Burkholderia plantarii, Burkholderia pyrrocinia, Burkholderia rhizoxinica, Burkholderia sacchari, Burkholderia sediminicola, Burkholderia soli, Burkholderia sordidicola, Burkholderia stabilis, Burkholderia terrae, Burkholderia terricola, Burkholderia thailandensis, Burkholderia tropica, Burkholderia tuberum, Burkholderia ubonensis, Burkholderia unamae, Burkholderia vietnamiensis*, and *Burkholderia xenovorans.*

The examples of the *Burkholderia* bacteria further include new bacteria of the genus *Burkholderia* newly separated from nature sources, such as *Burkholderia* sp. Preferable among these are *Burkholderia ambifaria, Burkholderia anthina, Burkholderia cenocepacia, Burkholderia cepacia, Burkholderia multivorans, Burkholderia pyrrocinia, Burkholderia stabilis, Burkholderia vietnamiensis,* and *Burkholderia* sp. Particularly, a strain of *Burkholderia* sp. YT (NITE P-613) and a strain of *Burkholderia multivorans* NBRC 102086 are preferable.

These *Burkholderia* bacteria are available from the following culture collections.

National Institute of Technology and Evaluation, Biotechnology Center, Biological Resource Center (NBRC) (2-5-8 Kazusakamatari, Kisarazu-shi, Chiba, 292-0818, Japan).
RIKEN BioResource Center, Japan Collection of Microorganisms (JCM) (2-1 Hirosawa, Wako, Saitama, 351-0198, Japan)
German Collection of Microorganisms and Cell Cultures GmbH (DSMZ) (Marschroder Weg 1b, D-38124 Brunswick, Germany)

The *Burkholderia* sp. YT strain is a microorganism found by Onodera et al. (Yamada-Onodera K. et at., Journal of Bioscience and Bioengineering (2007), Vol. 014, No. 5, pp. 416-419), which has been deposited with National Institute of Technology and Evaluation, Patent Microorganisms Depositary (2-5-8 Kazusakamatari, Kisarazu-shi, Chiba, 292-0818, Japan) on August 18, 2008 under the accession number NITE P-613.

### Polypeptide used in the present invention

Examples of the polypeptide used in the present invention include polypeptides having the amino acid sequence of SEQ ID NO:1 or SEQ ID NO:3 in the sequence listing (polypeptides (a1) and (b1)).

The polypeptide used in the present invention may be a polypeptide having an amino acid sequence derived from the amino acid sequence of SEQ ID NO:1 or SEQ ID NO:3 in the sequence listing by deletion, insertion, substitution and/or addition of one or a plurality of amino acid residues (polypeptides (a2) and (b2)). These polypeptides can be prepared in accordance with the known method described in documents such as Current Protocols in Molecular Biology (John Wiley and Sons, Inc., 1989), and any polypeptide capable of acting on 3-quinuclidinone and NADPH to form (R)-3-quinuclidinol and NADP is included in the polypeptide defined above.

The term "a plurality of amino acid residues" refers to, for example, not more than 35, preferably not more than 20, more preferably not more than 15, and still more preferably not more than 10, 9, 8, 7, 6, 5, 4, 3, or 2 amino acid residues.

The site or sites of the amino acid substitution, insertion, deletion and/or addition in the amino acid sequence of SEQ ID NO:1 or SEQ ID NO: 3 in the sequence listing is/are not particularly limited, but highly conserved regions should preferably be avoided. The term "highly conserved region" as used herein indicates a site at which multiple enzymes differing in origin, when compared upon optimal amino acid sequence alignment, show an identical amino acid sequence. The highly conserved regions can be determined by comparing the amino acid sequence of SEQ ID NO:1 with the amino acid sequences of known microorganism-derived alcohol dehydrogenases using such a tool as GENETYX.

The amino acid sequence modified by substitution, insertion, deletion and/or addition may contain only one type of modification (e.g. substitution) or two or more types of modifications (e.g. substitution and insertion) . In the case of substitution the substituting amino acid is preferably an amino acid similar in properties to the amino acid before substitution (amino acid of the same group). Those amino acids which belong to one and the same group among the groups given below are herein defined as amino acids of the same group.

(Group 1: neutral nonpolar amino acids) Gly, Ala, Val, Leu, Ile, Met, Cys, Pro, Phe
(Group 2: neutral polar amino acids) Ser, Thr, Gln, Asn, Trp, Tyr
(Group 3: acidic amino acids) Glu, Asp
(Group 4: basic amino acids) His, Lys, Arg

The polypeptide used in the present invention may be a polypeptide having a sequence identity of at least 85% with the amino acid sequence of SEQ ID NO:1 or SEQ ID NO:3 in the sequence listing and capable of acting on 3-quinuclidinone and NADPH to form (R)-3-quinuclidinol and NADP (polypeptides (a3) and (b3)).

The sequence identity with the amino acid sequence of SEQ NO:1 or SEQ ID NO:3 in the sequence listing is preferably not less than 90%, more preferably not less than 95%, still more preferably not less than 98%, and most preferably not less than 99%.

The sequence identity of the amino acid sequence of SEQ ID NO:1 in the sequence listing and the amino acid sequence of SEQ ID NO:3 in the sequence listing is 89.4%.

The amino acid sequence identity is expressed by the value obtained by comparing an amino acid sequence to be evaluated with the amino acid sequence of SEQ ID NO:1 or SEQ ID NO:3 in the sequence listing, dividing the number of sites where the amino acid residues in the two sequences are identical by the total number of the amino acid residues compared, and multiplying the resulting quotient by 100.

Further, an additional amino acid sequence may be linked to the amino acid sequence of SEQ ID NO:1 or SEQ ID NO:3, provided that the resulting polypeptide can act on 3-quinuclidinone and NADPH to form (R)-3-quinuclidinol and NADP. For example, a tag, such as a histidine tag or HA tag, may be added to the amino acid sequence. Alternatively, a fusion protein with another protein may also be produced. Further, peptide fragments may also be used as long as they can act on 3-quinuclidinone and NADPH to form (R)-3-quinuclidinol and NADP.

Even in the case that the polypeptide (a2), (a3), (b2), or (b3) acts on any NADPH to form (R)-3-quinuclidinol and NADP at a lower level, the polypeptide is included in the polypeptide in the present invention as long as the activity level is substantially the same. "Substantially the same activity level" indicates activity at the level of, for example, 50% to 100%, generally 70% to 100%, preferably 80% to 100%, and more preferably 90% or 95% to 100% of the original activity. The activity can be measured by the above method.

### DNA encoding polypeptide in the present invention

The DNA encoding the polypeptide in the present invention may be any As long as it can express the polypeptide in host cells harboring the DNA introduced therein by the later-described method, and the DNA may contain any untranslated region(s). The DNA may be a DNA or cDNA cloned from natural sources, or a DNA obtained by synthesis.

Once the polypeptide is obtained, a person skilled in the art will be able to obtain such a DNA from *Burkholderia* bacteria, the source of the polypeptide, by a known method such as the method shown below.

The gene manipulation procedures mentioned later herein, such as DNA cloning, vector preparation and transformation, can be carried out by the methods described in such a monograph as Molecular Cloning 2nd Edition (Cold Spring Harbor Laboratory Press, 1989), unless otherwise specified. The symbol "%" as used herein means "% (w/v)", unless otherwise specified.

First, the polypeptide used in the present invention, isolated by the method described above under "Polypeptide derived from bacteria of the genus *Burkholderia",* is digested with an appropriate endopeptidase, and the peptide fragments formed are isolated by reversed phase HPLC. The amino acid sequences of these peptide fragments are partly or wholly determined using, for example, a model ABI 492 protein sequencer (product of Applied Biosystems).

Based on the thus-obtained amino acid sequence information, PCR (Polymerase Chain Reaction) primers for amplifying a part of the DNA encoding the polypeptide are synthesized. Then, a chromosomal DNA of the microorganism which is the source of the polypeptide is prepared by a common DNA isolation technique such as the method of Visser et al. (Appl. Microbiol. Biotechnol., 53, 415 (2000)). This chromosomal DNA serving as a template, together with the above PCR primers, is used to amplify a part of the DNA encoding the polypeptide by PCR, and determine the base sequence thereof. The base sequence determination can be carried out using, for example, an Applied Biosystems 3130xl genetic analyzer (product of Applied Biosystems).

Once a part of the base sequence of the DNA encoding the polypeptide is found, the whole sequence can be determined, for example, by the Inverse PCR method (Nucl. Acids Ref., 16, 8186 (1988)).

Examples of the thus-obtained DNA in the present invention DNA encoding the polypeptide having the sequence of SEQ ID NO:1 in the sequence listing (i.e. DNA (A2)), and DNAs encoding the polypeptide having the sequence of SEQ ID NO:3 in the sequence listing (i.e. DNA (B2)). Specifically, the examples include the DNA of SEQ ID NO:2 in the sequence listing (i.e. DNA (A1)) and the DNA of SEQ ID NO:4 in the sequence listing (i.e. DNA (B1)).

The DNA used in the present invention may be a DNA hybridizing with a DNA complementary to the DNA of SEQ ID NO:2 or SEQ ID NO:4 in the sequence listing under stringent conditions and encoding a polypeptide capable of acting on 3-quinuclidinone and NADPH to form (R)-3-quinuclidinol and NADP (DNAs (A3) and (B3)).

The DNA used in the present invention may alternatively be a DNA having a sequence identity of at least 85% with the base sequence of SEQ ID NO:2 or SEQ ID NO:4 in the sequence listing and encoding a polypeptide capable of acting on 3-quinuclidinone and NADPH to form (R)-3-quinuclidinol and NADP (DNAs (A4) and (B4)).

The above statement includes the phrase "a DNA hybridizing with a DNA complementary to the DNA of SEQ ID NO:2 or SEQ ID NO:4 in the sequence listing under stringent conditions and encoding a polypeptide capable of acting on 3-quinuclidinone and NADPH to form (R)-3-quinuclidinol and NADP". Such a DNA means one that is obtainable by, for example, the colony hybridization, plaque hybridization or southern hybridization technique under stringent conditions using, as a probe, a DNA having a base sequence complementary to the base sequence of SEQ ID NO:2 or SEQ ID NO:4 in the sequence listing, and encodes a polypeptide capable of acting on 3-quinuclidinone and NADPH to form (R)-3-quinuclidinol and NADP.

The hybridization can be carried out according to the method described in Molecular Cloning, A laboratory manual, second edition (Cold Spring Harbor Laboratory Press, 1989), for instance. The "DNA hybridizing under stringent conditions" may be, for example, a DNA obtainable by carrying out hybridization using a filter with a colony- or plaque-derived DNA immobilized thereon at 65°C in the presence of 0.7 to 1.0 M NaCl, and then washing the filter using a two-fold concentrated SSC solution (composition of 1-fold concentrated SSC solution: 150 mM sodium chloride and 15 mM sodium citrate) at 65°c, preferably with a 0.5-fold concentrated SSC solution at 65°C, more preferably with a 0.2-fold concentrated SSC solution at 65°C, and still more preferably with a 0.1-fold concentrated SSC solution at 65°C.

The hybridization conditions are not particularly limited to those described above. Factors such as the temperature and salt concentrations are considered to influence the stringency of the hybridization, and a person skilled in the art can properly determine these factors to achieve the optimum astringency.

The DNA capable of hybridizing under the above conditions may be a DNA having a sequence identity with the DNA of SEQ ID NO:2 or SEQ ID NO:4 of not less than 70%, preferably not less than 85%, more preferably not less than 90%, still more preferably not less than 95%, and most preferably not less than 98%, and such a DNA is included in the above DNA as long as the polypeptide encoded is capable of acting on 3-quinuclidinone and NADPH to form (R)-3-quinuclidinol and NADP.

The "sequence identity (%)" herein is expressed by the value obtained by optimally aligning two DNAs to be compared, dividing the number of sites where the nucleic acid bases (e.g. A, T, C, G, U or I) in the two sequences are identical by the total number of bases compared, and then multiplying the resulting quotient by 100.

The following tools for sequence analysis, among others, can be used to calculate the sequence identity: GCG Wisconsin Package (Program Manual for The Wisconsin Package, Version 8, Sep., 1994, Genetics Computer Group, 575 Science Drive Madison, Wisconsin, USA 53711; Rice, P. (1996) Program Manual for EGCG Package, Peter Rice, The Sanger Centre, Hinxton Hall, Cambridge, CB10 1RQ, England); and the ExPASy World Wide Web molecular biology server (Geneva University Hospital and University of Geneva, Geneva, Switzerland).

### Recombinant organism capable of producing polypeptide used in the present invention

Examples of the recombinant organism capable of producing the polypeptide used in the present invention include recombinant organisms having the DNA (A1), (A2), (A3), (A4), (B1), (B2), (B3) or (B4).

The recombinant organism capable of producing the polypeptide used in the present invention can be obtained by inserting a DNA encoding the polypeptide used in the present invention into an expression vector to prepare a polypeptide expression vector, and transforming a host organism using the polypeptide expression vector. Culturing the recombinant organism on a medium where the recombinant organism can grow allows the recombinant organism to express the polypeptide in the present invention. Further, a method may also be utilized which includes introducing a polynucleotide encoding the polypeptide used in the present invention into a chromosome.

The expression vector to be used in the above procedure is not particularly limited so long as it allows the polypeptide encoded by the DNA to be expressed in an appropriate host organism. Examples of such a vector include plasmid vectors, phage vectors, and cosmid vectors. Further, shuttle vectors capable of gene exchange between different host strains can also be used.

In the case that the host is *Escherichia coli,* for instance, such a vector can suitably be used generally in the form of an expression vector containing an expression unit including such a regulatory factor as the lacUV5 promoter, trp promoter, trc promoter, tac promoter, lpp promoter, tufB promoter, recA promoter or pL promoter, operably linked to the DNA according to the present invention. Examples thereof include pUCN18 (see Example 2), pSTV28 (product of Takara Bio, Inc.) and pUCNT (WO 94/03613).

The term "regulatory factor" as used herein refers to a base sequence including a functional promoter and any related transcription element(s) (e.g. enhancer, CCAAT box, TATA box, SPI site, etc.).

The term "operably linked" as used herein refers to a condition in which various regulatory elements regulating the expression of the gene, such as a promoter and an enhancer, are linked to the gene in such a manner to be able to operate in host cells. It is well known to a person skilled in the art that the regulatory factor can vary in type and kind according to the host.

Those vectors, promoters and others which are utilizable in various organisms are described in detail in "Biseibutsugaku Kiso Koza 8: Idensikogaku (Basic Courses in Microbiology 8: Genetic Engineering), Kyoritsu Shuppan", for instance.

The host organism to be used for the expression of each polypeptide used in the present invention is not particularly limited so long as it is capable of being transformed with a polypeptide expression vector containing the DNA encoding the polypeptide and allows the expression of the polypeptide encoded by the DNA introduced. Preferably, the host organism is one for which host-vector systems have been developed.

Examples of microorganisms which can be used include bacteria such as those of the genera *Escherichia, Bacillus, Pseudomonas, Serratia, Brevibacterium, Corynebacterium,* Streptococcus, and *Lactobacillus*; actinomycetes such as those of the genera *Rhodococcus* and *Streptomyces*; yeasts such as those of the genera *Saccharomyces, Kluyveromyces, Schizosaccharomyces, Zygosaccharomyces,* Yarrowia, *Trichosporon, Rhodosporidium, Pichia,* and *Candida*; and fungi such as those of the genera Neurospora, *Aspergillus, Cephalosporium,* and *Trichoderma.*

In addition to the host-vector systems in microorganisms, various host-vector systems have also been developed in plants and animals and, in particular, systems for large-scale foreign protein expression using such insects as silkworms (Nature 315, 592-594 (1985)) or such plants as rape, maize and potato have been developed; these, too, can suitably be utilized. Among these, microorganisms are preferable from the introduction and expression efficiency viewpoint, bacteria are more preferable, and *Escherichia coli* (*E. coli*) is particularly preferable.

The polypeptide expression vector containing the DNA which encodes the polypeptide used in the present invention can be introduced into a host organism by a known method.

When, for example, polypeptide expression vector pNBS (see Preparation 1) prepared by introducing the DNA of SEQ ID NO:2 into an expression vector pUCN18 is introduced into *Escherichia coli* as the host microorganism, a recombinant organism, *E. coli* HB101 (pNBS) (see Preparation 4), resulting from the introduction of the vector into cells of the host, can be obtained by using commercially available *E. coli* HB101 competent cells (product of Takara Bio, Inc.), for instance, and carrying out the procedure according to the protocol attached thereto. Also in the case of a polypeptide expression vector pNBM (see Preparation 2) prepared by introducing the DNA of SEQ ID NO:4 into an expression vector pUCN18, a recombinant organism, *E. coli* HB101 (pNBM) (see Preparation 4), resulting from the introduction of the vector into cells of the host, can be obtained by the same procedure as above.

Also constructible is a recombinant organism in which two polypeptides, namely the polypeptide according to the present invention and a polypeptide capable of regenerating a reduced coenzyme as described below, both are expressed in one and the same cell. That is, a DNA encoding the polypeptide used in the present invention and a DNA encoding the polypeptide capable of regenerating a reduced coenzyme are inserted into the same vector, and the vector is introduced into the host cell. Examples of the recombinant organism obtained as above include *E. coli* HB101 (pNBSG) (see Preparation 4). The *E. coli* HB101 (pNBSG) is a recombinant organism constructed by introducing into *E. coli* HB101 a recombinant vector pNBSG (see Preparation 1) having two DNAs, namely the DNA of SEQ ID NO:2 and a DNA encoding a glucose dehydrogenase capable of regenerating a reduced coenzyme, introduced into an expression vector pUCN18.

Alternatively, a recombinant organism capable of producing two polypeptides, namely the polypeptide according to the present invention and the polypeptide capable of regenerating a reduced coenzyme, in one cell can be constructed by inserting a DNA encoding the polypeptide used in the present invention and a DNA encoding the polypeptide capable of regenerating a reduced coenzyme into two respective vectors belonging to different incompatibility groups, and then introducing them into one and the same host cell.

Examples of the recombinant organism obtained as above include *E. coli* HB101 (pNBS, pSTVG) (see Preparation 4). The *E. coli* HB101 (pNBS, pSTVG) is constructed by introducing the following two recombinant vectors into an *E. coli* HB101 competent cell (product of Takara Bio, Inc.): the recombinant vector pNBS (see Preparation 1) which is prepared by introducing the DNA of SEQ ID NO:2 into an expression vector pUCN18; and the recombinant vector pSTVG (see Preparation 3) which is prepared by introducing a DNA encoding a glucose dehydrogenase capable of regenerating a reduced coenzyme into a plasmid pSTV28 (product of Takara Bio, Inc.).

### production of (R)-3-quinuclidinol using polypeptide or recombinant organism

In the case of producing (R)-3-quinuclidinol by reducing 3-quinuclidinone with use of the above-mentioned polypeptide or a recombinant organism in which the polypeptide is expressed, the following non-limiting method may be employed.

To an appropriate solvent such as a 100 mM phosphate buffer (pH 6.5) are added 3-quinuclidinone or a salt thereof, a coenzyme such as NADPH or NADP⁺, and the culture of the recombinant organism and/or a treated product thereof or the like. The mixture is stirred and allowed to react at a controlled pH.

The term "treated product" as used herein means a material retaining the catalytic activity of the polypeptide, such as, for example, a cell disruption product, a crude extract, cultured cells, lyophilized cells, acetone-dried cells, a ground product of these, or a mixture of these. Here, the amount of the coenzyme to be used in the reaction can be greatly decreased by employing a recombinant organism capable of producing both the polypeptide according to the present invention and a polypeptide capable of regenerating a reduced coenzyme, such as *E. coli* HB101 (pNBSG) (see Preparation 4) and *E. coli* HB101 (pNBM, pSTVG) (see Preparation 4). The polypeptide capable of regenerating a reduced coenzyme will be described in detail later.

For the reaction, either 3-quinuclidinone itself or a salt thereof may be used. The salt may be an organic acid salt or an inorganic acid salt. Examples of an acid used for formation of the salt include inorganic acids such as hydrofluoric acid, hydrochloric acid, hydrobromic acid, hydroiodic acid, sulfuric acid, nitric acid, phosphoric acid, and boric acid; carboxylic acids such as formic acid, acetic acid, propionic acid, butanoic acid, pivalic acid, chloroacetic acid, trichloroacetic acid, trifluoroacetic acid, oxalic acid, L-tartaric acid, D-tartaric acid, and mandelic acid; and sulfonic acids such as methanesulfonic acid, trifluoromethanesulfonic acid, benzenesulfonic acid, p-toluenesulfonic acid, and camphorsulfonic acid. Preferable among these are hydrochloric acid, hydrobromic acid, sulfuric acid, acetic acid, pivalic acid, oxalic acid, L-tartaric acid, D-tartaric acid, mandelic acid, methanesulfonic acid, p-toluenesulfonic acid, and camphorsulfonic acid. Particularly, hydrochloric acid and sulfuric acid are preferable, and hydrochloric acid is most preferable.

The reaction is carried out at 5°C to 80°C, preferably 10°C to 60°C, and more preferably 20°C to 40°C, and the pH of the reaction mixture during the reaction is maintained at 3 to 10, preferably 4 to 9, and more preferably 5 to 8. The reaction can be carried out either batch-wise or in a continuous manner. In the case of a batch method, the substrate to be reacted may be added at a concentration of 0.01 to 100% (w/v), preferably 0.1 to 70%, and more preferably 0.5 to 50% of the total reaction mixture. An additional amount of the substrate may be further added during the reaction.

Here, it is effective to add a surfactant such as Triton (product of Nacalai Tesque, Inc.), Span (product of Kanto Chemical Co., Inc.), and Tween (product of Nacalai Tesque, Inc.) to the reaction mixture. In order to avoid inhibition of the reaction by the substrate and/or alcohol which is a product of the reduction reaction, an organic solvent insoluble in water, such as ethyl acetate, butyl acetate, isopropyl ether, toluene, and hexane, may be added to the reaction mixture. In order to increase the solubility of the substrate, an organic solvent soluble in water, such as methanol, ethanol, acetone, tetrahydrofuran, and dimethyl sulfoxide, can also be added.

The method of recovering (R)-3-quinuclidinol generated in the reduction reaction is not particularly limited, and (R)-3-quinuclidinol may be extracted, directly from the reaction mixture or after removal of the cells, with a solvent such as ethyl acetate, toluene, t-butyl methyl ether, hexane, n-butanol, or dichloromethane, and the extract is dehydrated and purified by, for example, distillation or silica gel column chromatography, whereby high-purity (R)-3-quinuclidinol can be easily obtained.

The (R)-3-quinuclidinol obtained by the present invention is 3-quinuclidinol in which the amount of (R)-3-quinuclidinol is larger than that of (S)-3-quinuclidinol. The optical purity thereof is usually not lower than 85% e.e., preferably not lower than 90% e.e., more preferably not lower than 95% e.e., still more preferably not lower than 97% e.e., and most preferably not lower than 99% e.e.

### Method for producing active pharmaceutical ingredient from optically active alcohol produced using polypeptide or transformant

A useful active pharmaceutical ingredient can be produced from (R)-3-quinuclidinol obtained by the above method. The useful active pharmaceutical ingredient is preferably one containing (R)-3-quinuclidinol as a building block, and solifenacin is particularly preferable. For example, (1S,3'R)-quinuclidin-3'-yl 1-phenyl-1,2,3,4-tetrahydroisoquinoline-2-carboxylate (solifenacin) can be' produced easily by a known method (e.g., the method taught in J. Med. Chem. 48, 6597-6606 (2005)).

### Polypeptide capable of regenerating reduced coenzyme

In the case of synthesizing (R)-3-quinuclidinol by reducing 3-quinuclidinone with a recombinant organism capable of producing the polypeptide used in the present invention, NADPH is required as a coenzyme. As described above, the reaction can be allowed to proceed just by adding a required amount of NADPH to the reaction system. However, the amount of the expensive coenzyme added can be greatly decreased by carrying out the reaction using the substrate, the polypeptide used in the present invention, in combination with an enzyme capable of converting an oxidized coenzyme (NADP⁺) into its reduced form NADPH (hereinafter, such a capability is referred to as the capability of regenerating a reduced coenzyme), that is, a coenzyme regenerating system. Examples of the enzyme capable of regenerating a reduced coenzyme include hydrogenase, formic acid dehydrogenase, alcoholic dehydrogenase, glucose-6-phosphate dehydrogenase, and glucose dehydrogenase. Suitably, glucose dehydrogenase is used.

Such a reaction can be performed by adding a coenzyme regenerating system to the asymmetric reduction reaction system. Alternatively, the reaction can be efficiently performed by employing as a catalyst a recombinant organism transformed by two DNAs, namely a DNA encoding the enzyme according to the present invention and a DNA encoding a polypeptide capable of regenerating a reduced coenzyme, which eliminates the need for separately preparing an enzyme capable of regenerating a reduced coenzyme and adding the enzyme to the reaction system. Such a recombinant organism can be obtained by the above method.

### EXAMPLES

The following examples illustrate the present invention in detail. They are, however, by no means limitative of the scope of the invention. Detailed procedures and so forth concerning the recombinant DNA technology used in the following examples are described in the following monographs:
Molecular Cloning 2nd Edition (Cold Spring Harbor Laboratory Press, 1989); and
Current Protocols in Molecular Biology (Greene Publishing Associates and Wiley-Interscience).

### (Preparation 1) Construction of recombinant vectors pNBS and pNBSG

### (Isolation of DNA encoding polypeptide capable of reducing 3-quinuclidinone derived from Burkholderia sp. YT)

A DNA encoding a polypeptide capable of reducing 3-quinuclidinone was obtained from the *Burkholderia* sp. YT strain (NITE P-613) by PCR (hereinafter, the polypeptide is referred to as RBS).

### (Preparation of chromosomal DNA of Burkholderia sp. YT)

A 500-ml Sakaguchi flask was charged with 50 ml of a liquid medium (pH 7) containing (per L) 16 g of bacto tryptone, 10 g of yeast extract, 5 g of sodium chloride, and 0.1 g of Adekanol LG-109 (product of NOF Corporation), and the flask was steam sterilized at 120°C for 20 minutes. The medium was inoculated with 5 ml of a culture fluid of the *Burkholderia* sp. YT strain which was obtained by the culturing on the same medium in advance. Then, culturing was carried out by shake culture at 30°C for 18 hours. From the resulting culture fluid, a chromosomal DNA was extracted according to the method of Murray et al. (Nucl. Acids Res., 8, 4321, 1980).

### (PCR reaction)

The chromosomal DNA of the *Burkholderia* sp. YT strain as a template was subjected to PCR with primer 1: 5'-ATATATACATATGACCCACCCGCAACCCCG-3' (SEQ ID NO:5 in the sequence listing), and primer 2: 5'-TATAGGTACCTTATCAAAGCTTGTCGAACGCGAG-3' (SEQ ID NO:6 in the sequence listing). As a result, a double stranded DNA (RBS gene) was obtained which had an NdeI recognition site added to the initiation codon site of the gene having the base sequence of SEQ ID NO:2 in the sequence listing, and had the termination codon immediately followed by a new termination codon (TAA) and a KpnI recognition site added. The PCR was performed using PrimeSTAR HS DNA polymerase (product of Takara Bio, Inc.) as a DNA polymerase under the reaction conditions recommended in the instruction manual.

### (Construction of recombinant vector pNBS)

The obtained RBS gene was digested with NdeI and KpnI, and the resulting gene was inserted into a plasmid pUCN18 (plasmid derived from pUC18 (product of Takara Bio, Inc.; GenBank Accession No. L09136) by converting the 185th base T to A by PCR for destruction of the NdeI site and converting the 471st-472nd bases GC to TG for introduction of a new NdeI site) at a site between the NdeI recognition site and KpnI recognition site downstream from the lac promoter. Thereby, a recombinant vector pNBS was constructed.

### (Construction of recombinant vector pNBSG further containing glucose dehydrogenase gene)

A plasmid pGDK1 (which can be obtained and prepared by the method described in Eur. J. Biochem., 186, 389 (1989)) as a template was subjected to PCR with primer 3: 5'-AAGGTACCCACACAGGAAACAACAATGTATAAAGATTTAGAAGGG-3' (SEQ ID NO:7 in the sequence listing), and primer 4 : 5'-ATATATCTAGATTATTATCCGCGTCCTGCTTGGAATGATGGGTAC-3' (SEQ ID NO:8 in the sequence listing). As a result, a double stranded DNA (GDH gene) was obtained which had the ribosome-binding site of *Escherichia coli* added at a site 5 bases upstream from the initiation codon of a glucose dehydrogenase (hereinafter referred to as GDH) gene derived from the Bacillus megaterium IAM 1030 strain; a KpnI recognition site added at a site just before the ribosome-binding site; and the termination codon immediately followed by an additional termination codon TAA and an XbaI recognition site added.

The obtained GDH gene was digested with KpnI and XbaI, and the resulting gene was inserted into a plasmid pNBS at a site between the KpnI recognition site and the XbaI recognition site downstream from the RBS gene. Thereby, a recombinant vector pNBSG was constructed.

### (Preparation 2) Construction of recombinant vector pNBM

### (Isolation of DNA encoding polypeptide capable of reducing 3-quinuclidinone derived from Burkholderia multivorans NBRC 102086)

A DNA encoding a polypeptide capable of reducing 3-quinuclidinone was obtained from the *Eurkholderia multivorans* NBRC 102086 strain by PCR (hereinafter, the polypeptide is referred to as RBM). A chromosomal DNA from the *Burkholderia multivorans* NBRC 102086 strain was prepared by the same procedure as that for preparation of a chromosomal DNA of the *Burkholderia* sp. YT strain.

### (PCR reaction)

The chromosomal DNA of the *Burkholderia multivorans* NBRC 102086 strain as a template was subjected to PCR with primer 5: 5'-ATATATACATATGCGCGCTGCACGCCCCCGTACG-3' (SEQ ID NO:9 in the sequence listing), and primer 6: 5'-TATAGGTACCTTATCACCGCGCGTCGAACGCGAGCGTC-3' (SEQ ID NO:10 in the sequence listing). As a result, a double stranded DNA (RBM gene) was obtained which had an NdeI recognition site added to the initiation codon site of the gene having the base sequence of SEQ ID NO:4 in the sequence listing, and had the termination codon immediately followed by a new termination codon (TAA) and a KpnI recognition site added. The PCR was performed using PrimeSTAR HS DNA polymerase (product of Takara Bio, Inc.) as a DNA polymerase under the reaction conditions recommended in the instruction manual.

### (Construction of recombinant vector pNBM)

The RBM gene was digested with NdeI and KpnI, and the resulting gene was inserted into the plasmid pUCN18 at a site between the NdeI recognition site and the KpnI recognition site downstream from the lac promoter. Thereby, a recombinant vector pNBM was constructed.

### (Preparation 3) (Construction of recombinant vector pSTVG

A plasmid pGDK1 (which can be obtained and prepared by the method described in Eur. J. Biochem., 186, 389 (1989)) as a template was subjected to PCR with primer 7: 5'-GCCGAATTCTAAGGAGGTTAACAATGTATAAAGATTTAGAAGG-3' (SEQ ID NO:11 in the sequence listing), and primer 8: 5'-CTGCAGGTCGACTTATCCGCGTCCTGCTTGG-3' (SEQ ID NO:12 in the sequence listing). As a result, a double stranded DNA (GDH gene) was obtained which had the ribosome-binding site of *Escherichia coli* added at a site 5 bases upstream from the initiation codon of a glucose dehydrogenase (hereinafter referred to as GDH) gene derived from the *Bacillus megaterium* IAM 1030 strain; an EcoRI recognition site added at a site just before the ribosome-binding site; and the termination codon immediately followed by SalI and PstI recognition sites added. The obtained GDH gene was digested with EcoRI and PstI, and the resulting gene was inserted into a plasmid pSTV28 (product of Takara Bio, Inc.) at the EcoRI-PstI site. Thereby, a recombinant vector pSTVG was constructed.

### (Preparation 4) Production of recombinant organism expressing polypeptide

*E. coli* HB101 competent cells (product of Takara Bio, Inc.) were transformed with the recombinant vector pNBS, so that a recombinant organism *E. coli* HB101 (pNBS) was obtained.

Similarly, another set of *E. coli* HB101 competent cells (product of Takara Bio, Inc.) was transformed with the recombinant vector pNBSG, so that a recombinant organism *E. coli* HB101 (pNBSG) was obtained.

Yet another set of *E. coli* HB101 competent cells (product of Takara Bio, Inc.) was transformed with two recombinant vectors, namely the recombinant vectors pNBS and pSTVG, so that a recombinant organism *E. coli* HB101 (pNBS, pSTVG) was obtained.

Yet another set of *E. coli* HB101 competent cells (product of Takara Bio, Inc.) was transformed with the recombinant vector pNBM, so that a recombinant organism *E. coli* HB101 (pNBM) was obtained.

Yet another set of *E. coli* HB101 competent cells (product of Takara Bio, Inc.) was transformed with two recombinant vectors, namely the recombinant vectors pNBM and pSTVG, so that a recombinant organism *E. coli* HB101 (pNBM, pSTVG) was obtained.

### (Preparation 5) Expression of polypeptide

Three recombinant organisms (*E. coli* HB101 (pNBS), *E. coli* HB101 (pNBSG), *E. coli* HB101 (pNBM))) and a recombinant organism *E. coli* HB101 (pUCN18) (comparative example) containing the vector plasmid pUCN18 were inoculated into respective 5-ml portions of 2 × YT medium (tryptone 1.6%, yeast extract 1.0%, NaCl 0.5%, (pH 7.0) containing 200 ug/ml of ampicillin, followed by 24 hours of shake culture at 37°C.

Also, two recombinant organisms (*E. coli* HB101 (pNBS, pSTVG) , *E. coli* HB101 (pNBM, pSTVG)) and a recombinant organism *E. coli* HB101 (pUCN18, pSTV28) (comparative example) containing the vector plasmids pUCN18 and pSTV28 were respective 5 portions of 2 × YT were inoculated into respective 5-ml portions of 2 × YT medium (tryptone 1.6%, yeast extract 1.0%, NaCl 0.5%, pH 7.0) containing 200 ug/ml of ampicillin and 100 ug/ml of chloramphenicol, followed by 24 hours of shake culture at 37°C.

### (Preparation 6) Reduction activity measurement

Cells in each of the culture fluids obtained through the above culturing were collected by centrifugation, and then suspended in 5 ml of a 100 mM phosphate buffer (pH 6.5) . The cells were disrupted using a model UH-50 ultrasonic homogenizer (product of SMT) and the cell debris was then removed by centrifugation to give a cell-free extract. The 3-quinuclidinone reduction activity and GDH activity of each of the cell-free extracts were measured.

The 3-quinuclidinone reduction activity was calculated by adding 3-quinuclidinone (10 mM), a coenzyme NADPH (0.25 mM), and a crude enzyme liquid to a 100 mM phosphate buffer (pH 6.5), allowing the reaction to proceed at 30°C for one minute, and then determining the rate of decrease in the absorbance at a wavelength of 340 nm. The enzyme activity of oxidizing 1 µmol of NADPH to NADP in one minute under these reaction conditions was defined as 1 U.

The GDH activity was calculated by adding glucose (0.1 M), a coenzyme NADP (2 mM), and a crude enzyme liquid to a 1-M Tris-hydrochloride buffer (pH 8.0), allowing the reaction to proceed at 25°C for one minute, and then determining the rate of increase in the absorbance at a wavelength of 340 nm. The enzyme activity of reducing 1 µmol of NADP to NADPH in one minute under these reaction conditions was defined as 1 U.

The 3-quinuclidinone reduction activity and GDH activity of the recombinant organisms are listed below.

*E. coli* HB101 (pUCN18) and *E. coli* HB101 (pUCN18, pSTV28) (both are comparative examples) showed both 3-quinuclidinone reduction activity and GDH activity of not higher than 0.1 U/mg.

*E. coli* HB101 (pNBS) in which only RBS was expressed showed 3-quinuclidinone reduction activity of 100 U/mg. *E. coli* HB101 (pNBSG) in which RBS and GDH were coexpressed showed 3-quinuclidinone reduction activity of 60 U/mg and GDH activity of 440 U/mg. *E, coli* HB101 (pNBS, pSTVG) in which RBS and GDH were coexpressed showed 3-quinuclidinone reduction activity of 120 U/mg and GDH activity of 120 U/mg.

*E. coli* HB101 (pNBM) in which only RBM was expressed showed 3-quinuclidinone reduction activity of 40 U/mg. *E. coli* HB101 (pNBM, pSTVG) in which RBM and GDH were coexpressed showed 3-quinuclidinone reduction activity of 40 U/mg and GDH activity of 140 u/mg.

As above, each and every one of the five recombinant organisms had 3-quinuclidinone reduction activity and expressed RBS or RUM. Each of *E. coli* HB101 (pNBSG), *E. coli* HB101 (pNBS, pSTVG), and *E. coli* HB101 (pNBM, pSTVG), which contain a GDH gene, expressed GDH.

### (Example 1) Production of (R)-3-quinuclidinol using recombinant organism E. coli HB101 (pNBS)

*E. coli* HB101 (pNBS) was cultured under the conditions described in Preparation 5, and then cells were disrupted using an ultrasonic homogenizer to give 100 ml of a cell-free extract. To the cell-free extract (100 ml) were added glucose dehydrogenase (700 U, trade name: GLUCDH "Amano" II, product of Amano Enzyme Inc.), 7 g of glucose, 3 mg of NADP⁺, and 5 g of 3-quinuclidinone hydrochloride. The mixture was stirred at 30°C for 24 hours while the pH was adjusted to 6.5 by dropping of a 5 N aqueous solution of sodium hydroxide. A portion of the reaction mixture was sampled, adjusted with 6 N sodium hydroxide to have a pH of not lower than 13, and then extracted with n-butanol. The extract was analyzed under the following condition (1) for gas chromatography to measure the rate of conversion to (R)-3-quinuclidinol. Also, the optical purity of (R)-3-quinuclidinol was measured by analyzing the extract under the following condition (2) for gas chromatography. The measured rate of conversion to (R)-3-quinuclidinol was 98%, and the measured optical purity thereof was not lower than 99% e.e.

### [Analysis condition (1) for gas chromatography]

Column: Rtx-5 amine capillary column (0.25 mm (inner diameter) × 30 m; product of Restek)
Detector: hydrogen flame ionization detector
Injection site temperature: 220°C, column temperature: 150°C
Detector temperature: 220°C
Carrier gas: helium, flow rate = 130 KPa

### [Analysis condition (2) for gas chromatography]

Column: γ-DEX 225 capillary column (0.25 mm (inner diameter) × 30 m; product of Supelco)
Detector: hydrogen flame ionization detector
Injection site temperature: 220°C, column temperature: 150°C
Detector temperature: 220°C
Carrier gas: helium, flow rate = 70 KPa

### (Example 2) Production of (R)-3-quinuclidinol using recombinant organism E. coli HB101 (pNBSG)

*E. coli* HB101 (pNBSG) was cultured under the conditions described in Preparation 5 to give 100 ml of a culture fluid. To the culture fluid (100 ml) were added 7 g of glucose, 3 mg of NADP⁺, and 5 g of 3-quinuclidinone hydrochloride. The mixture was stirred at 30°C for 24 hours while the pH was adjusted to 6.5 by dropping of a 5 N aqueous solution of sodium hydroxide. A portion of the reaction mixture was sampled, and then treated and analyzed by the same procedures as those in Example 1, so that the rate of conversion to (R)-3-quinuclidinol and the optical purity of the product were measured. The measured rate of conversion was not lower than 99%, and the measured optical purity was not lower than 99% e.e.

### (Example 3) Production of (R)-3-quinuclidinol using recombinant organism E. coli HB101 (pNBS, pSTVG)

*E. coli* HB101 (pNBS, pSTVG) was cultured under the conditions described in Preparation 5 to give 100 ml of a culture fluid. To the culture fluid (100 ml) were added 7 g of glucose, 3 mg of NADP⁺, and 5 g of 3-quinuclidinone hydrochloride. The mixture was stirred at 30°C for 24 hours while the pH was adjusted to 6.5 by dropping of a 5 N aqueous solution of sodium hydroxide. A portion of the reaction mixture was sampled, and then treated and analyzed by the same procedures as those in Example 1, so that the rate of conversion to (R)-3-quinuclidinol and the optical purity of the product were measured. The measured rate of conversion was not lower than 99%, and the measured optical purity was not lower than 99% e.e.

### (Example 4) Production of (R)-3-quinuclidinol using recombinant organism E. coli HB101 (pNBM)

*E. coli* HB101 (pNBM) was cultured under the conditions described in Preparation 5, and then cells were disrupted using an ultrasonic homogenizer to give 100 ml of a cell-free extract. To the cell-free extract (100 ml) were added glucose dehydrogenase (700 U, trade name: GLUCDH "Amano" II, product of Amano Enzyme Inc.), 7 g of glucose, 3 mg of NADP⁺, and 5 g of 3-quinuclidinone hydrochloride. The mixture was stirred at 30°C for 24 hours while the pH was adjusted to 6.5 by dropping of a 5 N aqueous solution of sodium hydroxide. A portion of the reaction mixture was sampled, and then treated and analyzed by the same procedures as those in Example 1, so that the rate of conversion to (R)-3-quinuclidinol and the optical purity of the product were measured. The measured rate of conversion was not lower than 99%, and the measured optical purity was not lower than 99% e.e.

### (Example 5) Production of (R)-3-quinuclidinol using recombinant organism E. coli HB101 (pNBM, pSTVG)

*E. coli* HB101 (pNBM, pSTVG) was cultured under the conditions described in Preparation 5 to give 100 ml of a culture fluid. To the culture fluid (100 ml) were added 7 g of glucose, 3 mg of NADP⁺, and 5 g of 3-quinuclidinone hydrochloride. The mixture was stirred at 30°C for 24 hours while the pH was adjusted to 6.5 by dropping of a 5 N aqueous solution of sodium hydroxide. A portion of the reaction mixture was sampled, and then treated and analyzed by the same procedures as those in Example 1, so that the rate of conversion to (R)-3-quinuclidinol and the optical purity of the product were measured. The measured rate of conversion was not lower than 99%, and the measured optical purity was not lower than 99% e.e.

### (Example 6) Production of (R)-3-quinuclidinol using recombinant organism E. coli HB101 (pNBS, pSTVG)

*E. coli* HB101 (pNBS, pSTVG) was cultured under the conditions described in Preparation 5 to give 300 ml of a culture fluid. To the culture fluid (300 ml) were added 57 g of glucose, 30 mg of NADP⁺, and 46.5 g of 3-quinuclidinone hydrochloride. The mixture was stirred at 30°C for 40 hours while the pH was adjusted to 6.5 by dropping of a 5 N aqueous solution of sodium hydroxide. After completion of the reaction, the reaction mixture was adjusted with 10 N NaOH to have a pH of 13, and extracted three times with n-butanol in an amount twice the amount of the reaction mixture. The solvent was removed under reduced pressure, and the residue was dried to give 34.2 g of (R)-3-quinuclidinol crystals. The (R)-3-quinuclidinol crystals were analyzed by the same method as that in Example 1, and the measured optical purity was not lower than 99% e.e.

### (Example 7) Production of (1S,3'R)-quinuclidin-3'-yl 1-phenyl-1,2,3,4-tetrahydroisoquinoline-2-carboxylate hydrochloride

### (Step 1)

To 200 ml of dichloromethane were added 10.6 g of (S)-1-phenyl-1,2,3,4-tetrahydroisoquinoline and 10.6 g of potassium carbonate. To the mixture, while being cooled in ice, was further added a mixed solution of 20 ml of dichloromethane and 5.8 ml of ethyl chloroformate. The mixture was then stirred at room temperature overnight. Thereafter, water was added to the reaction mixture, and the resulting mixture was stirred at room temperature for 30 minutes. The organic layer was separated from the mixture, washed with water and saturated saline, dehydrated with magnesium sulfate, and vacuum concentrated. The concentrate was purified by silica gel chromatography, whereby (S)-1-phenyl-1, 2,3,4-tetrahydroisoquinoline-2-carboxylic acid was obtained.

### (Step 2)

An amount of 12 g of the purified (S)-1-phenyl-1,2,3,4-tetrahydroisoquinoline-2-carboxylic acid and 16.3 g of (R)-3-quinuclidinol obtained by the method in Example 6 were added to 120 ml of toluene. The mixture was then mixed with 1.7 g of sodium hydride, and then heated at 140°C for three hours. After completion of the reaction, the reaction mixture was cooled to room temperature, mixed with saturated saline, and extracted with ethyl acetate. The extract was washed with water, and then extracted with 20% hydrochloric acid. The aqueous layer was adjusted with 1 M sodium hydroxide to have a pH of 10, and then extracted with ethyl acetate. The organic layer was washed with saturated saline, and dehydrated with magnesium sulfate. The extract was vacuum concentrated, and crystallized in acetonitrile and diethyl ether to which hydrochloric acid was added, whereby 10.1 g of (1S,3'R)-quinuclidin-3'-yl 1-phenyl-1,2,3,4-tetrahydroisoquinoline-2-carboxylate hydrochloride was obtained in the form of colorless crystals.
As above, the active pharmaceutical ingredient (1S,3'R)-quinuclidin-3'-yl 1-phenyl-1,2,3,4-tetrahydroisoquinoline-2-carboxylate hydrochloride (solifenacin hydrochloride) was easily produced from the produced (R)-3-quinuclidinol.

## Claims

1. A method for producing (R)-3-quinuclidinol, comprising reducing 3-quinuclidinone or a salt thereof in the presence of a polypeptide, an organism capable of producing the polypeptide, or a treated product of the organism,
the polypeptide being selected from the group consisting of :
(a1) a polypeptide having an amino acid sequence of SEQ ID NO:1 in the sequence listing;
(a2) polypeptides having an amino acid sequence derived from the amino acid sequence of SEQ ID NO:1 in the sequence listing by substitution, deletion, insertion and/or addition of one or a plurality of amino acid residues and capable of acting on 3-quinuclidinone and NADPH to form (R)-3-quinuclidinol and NADP;
(a3) polypeptides having a sequence identity of at least 85% with the amino acid sequence of SEQ ID NO:1 in the sequence listing and capable of acting on 3-quinuclidinone and NADPH to form (R)-3-quinuclidinol and NADP;
(b1) a polypeptide having an amino acid sequence of SEQ ID NO:3 in the sequence listing;
(b2) polypeptides having an amino acid sequence derived from the amino acid sequence of SEQ ID NO:3 in the sequence listing by substitution, deletion, insertion and/or addition of one or a plurality of amino acid residues and capable of acting on 3-quinuclidinone and NADPH to form (R)-3-quinuclidinol and NADP; and
(b3) polypeptides having a sequence identity of at least 85% with the amino acid sequence of SEQ ID NO:3 in the sequence listing and capable of acting on 3-quinuclidinone and NADPH to form (R)-3-quinuclidinol and NADP.

2. The production method according to claim 1, wherein the organism capable of producing the polypeptide selected from the group consisting of (a1), (a2), (a3), (b1), (b2), and (b3) is a recombinant organism having introduced therein a DNA selected from the group consisting of:
(A1) a DNA of SEQ ID NO:2 in the sequence listing;
(A2) DNAs encoding the polypeptide having the amino acid sequence of SEQ ID NO:1 in the sequence listing;
(A3) DNAs hybridizing with a DNA complementary to the DNA of SEQ ID NO:2 in the sequence listing under stringent conditions and encoding a polypeptide capable of acting on 3-quinuclidinone and NADPH to form (R)-3-quinuclidinol and NADP;
(A4) DNAs having a sequence identity of at least 85% with the base sequence of SEQ ID NO:2 in the sequence listing and encoding a polypeptide capable of acting on 3-quinuclidinone and NADPH to form (R)-3-quinuclidinol and NADP;
(B1) a DNA of SEQ ID NO:4 in the sequence listing;
(B2) DNAs encoding the polypeptide having the amino acid sequence of SEQ ID NO:3 in the sequence listing;
(B3) DNAs hybridizing with a DNA complementary to the DNA of SEQ ID NO:4 in the sequence listing under stringent conditions and encoding a polypeptide capable of acting on 3-quinuclidinone and NADPH to form (R)-3-quinuclidinol and NADP; and
(B4) DNAs having a sequence identity of at least 85% with the base sequence of SEQ ID NO:4 in the sequence listing and encoding a polypeptide capable of acting on 3-quinuclidinone and NADPH to form (R)-3-quinuclidinol and NAP.

3. The production method according to claim 2,
wherein the recombinant organism is *Escherichia coli.*

4. The production method according to any one of claims 1 to 3, further comprising
utilizing a polypeptide capable of regenerating a reduced coenzyme.

5. The production method according to claim 4,
wherein the polypeptide is produced by the recombinant organism capable of producing the polypeptide capable of regenerating a reduced coenzyme.

6. The production method according to claim 4 or 5,
wherein the polypeptide capable of regenerating a reduced coenzyme is a glucose dehydrogenase.

7. A method for producing (R)-3-quinuclidinol, comprising
reducing 3-quinuclidinone or a salt thereof in the presence of a polypeptide derived from bacteria of the genus *Burkholderia.*

8. A method for producing an active pharmaceutical ingredient, comprising the step of
producing (R)-3-quinuclidinol by reducing 3-quinuclidinone or a salt thereof in the presence of a polypeptide, an organism capable of producing the polypeptide, or a treated product of the organism,
the polypeptide being selected from the group consisting of:
(a1) a polypeptide having an amino acid sequence of SEQ ID NO:1 in the sequence listing;
(a2) polypeptides having an amino acid sequence derived from the amino acid sequence of SEQ ID NO:1 in the sequence listing by substitution, deletion, insertion and/or addition of one or a plurality of amino acid residues and capable of acting on 3-quinuclidinone and NADPH to form (R)-3-quinuclidinol and NADP;
(a3) polypeptides having a sequence identity of at least 85% with the amino acid sequence of SEQ ID NO:1 in the sequence listing and capable of acting on 3-quinuclidinone and NADPH to form (R)-3-quinuclidinol and NADP;
(b1) a polypeptide having an amino acid sequence of SEQ ID NO:3 in the sequence listing;
(b2) polypeptides having an amino acid sequence derived from the amino acid sequence of SEQ ID NO:3 in the sequence listing by substitution, deletion, insertion and/or addition of one or a plurality of amino acid residues and capable of acting on 3-quinuclidinone and NADPH to form (R)-3-quinuclidinol and NADP; and
(b3) polypeptides having a sequence identity of at least 85% with the amino acid sequence of SEQ ID NO:3 in the sequence listing and capable of acting on 3-quinuclidinone and NADPH to form (R)-3-quinuclidinol and NADP.

9. The production method according to claim 8,
wherein the active pharmaceutical ingredient is solifenacin.


SEQUENCE LISTING

<110> KANEKA CORPORATION
<110> NATIONAL UNIVERSITY CORPORATION NARA INSTITUTE OF SCIENCE AND
TECHNOLOGY

<120> PROCESS FOR PRODUCTION OF (R)-3-QUINUCLIDINOL

<130> T2992 EP S3

<140> EP 10 76 7119.0

<141> 2010-04-22

<150> JP 2009-105390

<151> 2009-04-23

<160> 12

<170> PatentIn version 3.1

<210> 1
<211> 263
<212> PRT
<213> Burkholderia sp.

<400> 1

Met Thr His Pro Gln Pro Arg Thr Ile Val Ile Thr Gly Ala Gly Thr
1               5                   10                  15

Gly Ile Gly Ala Ala Cys Ala Arg Arg Phe Ala Arg Arg Gly Asp Arg
            20                  25                  30

Val Val Leu Ile Gly Arg Arg Gln Ala Pro Leu Asp Ala Leu Ala Ala
        35                  40                  45

Glu Thr Gly Gly Leu Ala Leu Ala Gly Asp Ala Ala Ser Thr Ala Asp
    50                  55                  60

Trp Ala Gly Phe Leu Pro Arg Ile Ala Glu Arg Phe Gly Arg Val Asp
65                  70                  75                  80

Ala Leu Val Ala Cys Ala Gly Gly His Gly Leu Gly Arg Ala Asp Glu
                85                  90                  95

Thr Asp Asp Ala Gln Trp Arg Asp Ala Met His Ala Asn Leu Asp Thr
            100                 105                 110

Ala Phe Val Ser Ala Arg Ala Cys Leu Pro Asp Leu Ile Ala Gln Arg
        115                 120                 125

Gly Ser Ile Val Leu Val Ala Ser Ile Ala Ala Leu Ala Ala Gly Pro
    130                 135                 140

Ser Val Cys Gly Tyr Thr Val Gly Lys His Ala Leu Leu Gly Leu Ala
145                 150                 155                 160

Arg Ser Leu Ala Arg Asp Tyr Gly Pro His Gly Val Arg Ala Asn Ala
                165                 170                 175

Val Cys Pro Gly Trp Val Arg Thr Pro Met Ala Asp Ala Glu Met Glu
            180                 185                 190

Pro Leu Met Ser Ala His Gly Asp Thr Leu Asp Gly Ala Tyr Ala Arg
        195                 200                 205

Val Ser Ala Asp Val Pro Leu Arg Arg Ala Ala Asp Pro Asp Glu Ile
    210                 215                 220

Ala Ala Val Cys Ala Phe Leu Thr Ser Pro Asp Ala Ser Phe Val Thr
225                 230                 235                 240

Gly Ala Thr Leu Val Ala Asp Gly Gly Ala Met Val Val Asp Val Pro
                245                 250                 255

Thr Leu Ala Phe Asp Lys Leu
            260

<210>  2
<211>  792
<212>  DNA
<213>  Burkholderia sp.

<400>  2
atgacccacc cgcaaccccg cacgatcgtg atcaccggcg cgggcaccgg tatcggcgcc      60

gcgtgcgcgc gccgcttcgc ccgccgcggc gaccgcgtcg tgctgatcgg gcggcgccag     120

gcgccgctcg acgcgctggc cgccgaaacg ggcggcctcg cgctggccgg cgatgccgcg     180

agcaccgccg actgggccgg cttcctgccg cggatcgccg aacgcttcgg ccgcgtcgac     240

gcgctcgtcg cgtgcgcggg cggccacggc ctcggccgcg cggacgaaac cgacgacgcg     300

caatggcgcg acgcgatgca cgcgaacctc gacaccgcgt tcgtcagcgc gcgcgcgtgc     360

ctgcccgacc tgatcgcgca gcgcggcagc atcgtgctgg tcgcgtcgat cgccgcgctc     420

gcggccggcc ccagcgtgtg cggctatacg gtcggcaagc atgcgctgct cgggctcgcg     480

cggtcgctcg cacgcgacta cgggccgcac ggcgtgcggg cgaacgcggt atgccccggc     540

tgggtccgca cgccgatggc cgacgcggag atggagccgc tgatgtcggc acacggcgac     600

acgctcgacg gtgcgtatgc gcgcgtcagc gccgacgtgc cgctgcggcg cgcggcagac     660

ccggacgaga tcgcggccgt gtgcgcgttc ctcacgtcgc cggacgcgtc tttcgtgacc     720

ggcgcgacgc tcgtcgccga cggcggcgcg atggtcgtcg acgtgccgac gctcgcgttc     780

gacaagcttt ga                                                         792

<210>  3
<211>  263
<212>  PRT
<213>  Burkholderia multivorans

<400>  3

Met Arg Ala Ala Arg Pro Arg Thr Ile Val Ile Thr Gly Ala Gly Thr
1               5                   10                  15

Gly Ile Gly Ala Ala Cys Ala Arg Arg Phe Ala Arg Gly Gly Asp Arg
            20                  25                  30

Val Val Leu Ile Gly Arg Arg Pro Ala Pro Leu Glu Ala Leu Ala Ala
        35                  40                  45

Glu Thr Gly Gly Leu Ala Leu Ala Gly Asp Ala Ala Gly Thr Ala Asp
    50                  55                  60

Trp Ala Arg Phe Val Pro Ala Ile Ala Thr Arg Val Gly Arg Ile Asp
65                  70                  75                  80

Ala Leu Ile Ala Cys Ala Gly Gly His Gly Val Gly Arg Ala Asp Glu
                85                  90                  95

Thr Gly Asp Ala Gln Trp Arg Asp Ala Met His Ala Asn Leu Asp Thr
            100                 105                 110

Ala Phe Val Ser Ala Arg Ala Cys Leu Pro Asp Leu Val Ala Gln Arg
        115                 120                 125

Gly Cys Ile Val Leu Val Ala Ser Ile Ala Ala Leu Ala Ala Gly Pro
    130                 135                 140

Gly Val Cys Gly Tyr Thr Val Gly Lys His Ala Leu Leu Gly Leu Ala
145                 150                 155                 160

Arg Ser Leu Ala Arg Asp Tyr Gly Pro His Gly Val Arg Ala Asn Ala
                165                 170                 175

Val Cys Pro Gly Trp Val Arg Thr Pro Met Ala Asp Ala Glu Met Gln
            180                 185                 190

Pro Leu Met Asp Ala His Gly Asp Thr Leu Glu Arg Ala Tyr Ala Arg
        195                 200                 205

Val Ser Ala Asp Val Pro Leu Arg Arg Ala Ala Asp Pro Asp Glu Ile
    210                 215                 220

Ala Ala Val Cys Ala Phe Leu Ala Ser Pro Asp Ala Ser Phe Val Thr
225                 230                 235                 240

Gly Ala Thr Leu Val Ala Asp Gly Gly Ala Ser Val Val Asp Val Pro
                245                 250                 255

Thr Leu Ala Phe Asp Ala Arg
            260

<210>  4
<211>  792
<212>  DNA
<213>  Burkholderia multivorans

<400>  4
atgcgcgctg cacgcccccg tacgatcgtc attaccggcg ccggcaccgg catcggcgcc      60

gcctgcgcac gccgtttcgc gcgcggcggc gaccgtgtcg tgctgatcgg ccggcggccc     120

gcgccgctcg aggcgctggc cgccgaaacg ggcggcctcg cgctcgcggg cgacgcggcc     180

ggcacggccg actgggcacg cttcgtgccc gcgatcgcca cgcgcgtcgg ccgcatcgac     240

gcgttgatcg cgtgcgcggg cgggcacggc gtcggccgcg cggacgaaac cggcgacgcg     300

cagtggcgcg acgccatgca tgcgaacctc gacaccgcat tcgtcagtgc acgcgcatgc     360

ctgccggacc tcgtcgcgca gcgcggctgc atcgtgctcg tggcgtcgat cgcggcgctc     420

gcggccggcc ccggcgtgtg cggctacacg gtcggcaagc atgcgctgct cggcctcgcg     480

cgctcgctcg cgcgcgacta cgggccgcac ggcgtgcgcg cgaacgcggt atgcccgggc     540

tgggtccgca cgccgatggc cgacgcggaa atgcagccgc tgatggacgc acacggcgac     600

acgctcgagc gcgcctacgc gcgcgtgagc gccgacgtgc cgctgcggcg cgcggcggac     660

cccgacgaga tcgcggcggt ctgcgcattt ctggcgtcgc cggacgcgtc gttcgtgacg     720

ggcgcgacgc tggtcgcgga cggcggcgcg agcgtcgtcg acgtgccgac gctcgcgttc     780

gacgcgcggt ga                                                         792

<210>  5
<211>  30
<212>  DNA
<213>  Artificial sequence

<220>
<221>  source
<223>  /note= "Description of artificial sequence: primer"

<400>  5
atatatacat atgacccacc cgcaaccccg                                       30

<210>  6
<211>  34
<212>  DNA
<213>  Artificial sequence

<220>
<221>  source
<223>  /note= "Description of artificial sequence: primer"

<400>  6
tataggtacc ttatcaaagc ttgtcgaacg cgag                                    34


<210>  7
<211>  45
<212>  DNA
<213>  Artificial sequence

<220>
<221>  source
<223>  /note= "Description of artificial sequence: primer"

<400>  7
aaggtaccca cacaggaaac aacaatgtat aaagatttag aaggg                        45


<210>  8
<211>  45
<212>  DNA
<213>  Artificial sequence

<220>
<221>  source
<223>  /note= "Description of artificial sequence: primer"

<400>  8
atatatctag attattatcc gcgtcctgct tggaatgatg ggtac                        45


<210>  9
<211>  34
<212>  DNA
<213>  Artificial sequence

<220>
<221>  source
<223>  /note= "Description of artificial sequence: primer"

<400>  9
atatatacat atgcgcgctg cacgcccccg tacg                                    34


<210>  10
<211>  38
<212>  DNA
<213>  Artificial sequence

<220>
<221>  source
<223>  /note= "Description of artificial sequence: primer"

<400>  10
tataggtacc ttatcaccgc gcgtcgaacg cgagcgtc                                38


<210>  11
<211>  43
<212>  DNA
<213>  Artificial sequence

<220>
<221>  source
<223>  /note= "Description of artificial sequence: primer"

<400>  11
gccgaattct aaggaggtta acaatgtata aagatttaga agg                    43


<210>  12
<211>  31
<212>  DNA
<213>  Artificial sequence

<220>
<221>  source
<223>  /note= "Description of artificial sequence: primer"

<400>  12
ctgcaggtcg acttatccgc gtcctgcttg g                                 31